# DEMANDE DE BREVET EUROPEEN

(11) **EP 2 505 172 A1**
(43) Date de publication de la demande: **03.10.2012**
(21) Numéro de dépôt: 12162390.4
(22) Date de dépôt: 30.03.2012
(51) Int. Cl.: A61F 13/38

(54) **Bâtonnet ouaté à embouts de sécurité**

(30) Priorité: 31.03.2011 FR 1152745
(71) Demandeur: Groupe Lemoine, 92100 Boulogne Billancourt (FR)
(72) Inventeur: Lemoine, Philippe, 75016 PARIS (FR)
(74) Mandataire: Delprat, Olivier

(57) **Abrégé**

Bâtonnet ouaté comprenant un bâtonnet (1) et au moins un embout (2) monté à l'une des extrémités du bâtonnet (1).

L'embout (2) comporte au moins une dimension transversale supérieure à celle d'un conduit auditif d'un utilisateur de manière à limiter son insertion dans le conduit auditif

## Description

L'invention concerne les embouts de bâtonnet ouaté, et plus particulièrement la protection d'un conduit auditif contre une introduction d'un embout de bâtonnet ouaté lors de son utilisation.

Un bâtonnet ouaté est un bâtonnet dont les deux extrémités sont recouvertes d'une boule d'ouate de cellulose de manière à former un embout absorbant.

Ces bâtonnets permettent de nettoyer une oreille en la débarrassant de l'excès de cérumen qu'elle secrète. Ils permettent également d'absorber l'eau qui s'est accumulée au niveau du pavillon de l'oreille, à l'entrée du conduit auditif, et qui est susceptible de glisser dans le conduit auditif et boucher l'oreille.

Cependant, il est recommandé par les médecins que les embouts ouatés des bâtonnets ne soient utilisés que pour nettoyer le pavillon de l'oreille et qu'ils ne soient pas introduits profondément dans le conduit auditif.

En effet, le cérumen sécrété dans le conduit auditif joue un rôle important dans la protection du canal auditif.

En premier lieu, les cellules de cérumen formées dans le centre de la membrane du tympan migrent vers l'extérieur depuis l'ombilic du tympan jusqu'aux parois du canal de l'oreille puis jusqu'à l'entrée du canal auditif, le cérumen entraînant ainsi avec lui toute les poussières, saletés et particules de matières qui pourraient s'accumuler dans le canal.

En second lieu, le cérumen permet la lubrification du canal auditif, empêchant ainsi un assèchement et des démangeaisons de la peau à l'intérieur du canal.

Enfin, le cérumen joue également un rôle antibactérien et antifongique grâce, principalement, à la présence d'acides gras saturés, de lysozyme et au pH relativement acide du cérumen.

Un embout de bâtonnet ouaté, s'il est introduit trop profondément dans le canal auditif peut alors repousser le cérumen vers le tympan, créant éventuellement un bouchon de cérumen, et empêchant toutes les saletés d'être évacuées du conduit auditif.

Par ailleurs, un embout de bâtonnet ouaté introduit profondément dans un conduit auditif peut causer une otite externe, et son usage intempestif peut provoquer des réactions inflammatoires d'irritation, et parfois même, de façon accidentelle, des perforations tympaniques.

L'invention permet de pallier les problèmes mentionnés ci-dessus en proposant un bâtonnet ouaté de sécurité permettant de prévenir son introduction profonde à l'intérieur d'un conduit auditif, notamment d'un adulte.

L'invention a donc pour objet un bâtonnet ouaté comprenant un bâtonnet et au moins un embout monté à l'une des extrémités du bâtonnet.

Un autre but de l'invention est de proposer un bâtonnet ouaté ayant des propriétés d'absorption améliorées.

Selon une caractéristique générale du bâtonnet ouaté, l'embout comporte au moins une dimension transversale supérieure à celle d'un conduit auditif d'un utilisateur de manière à limiter son insertion dans le conduit auditif.

De préférence, ladite dimension transversale est comprise entre 5 mm et 9 mm, et plus particulièrement entre 5,7 mm et 9 mm.

Selon une autre caractéristique de l'embout, celui-ci a une section longitudinale globalement ovale définie par un grand axe s'étendant selon l'axe du bâtonnet et un petit axe ayant ladite dimension comprise entre 5 mm et 9 mm, et plus particulièrement entre 5,7 mm et 9 mm.

Avantageusement, les moyens d'absorption peuvent comprendre entre 60 mg et 120 mg de ouate de cellulose, et notamment 82 mg de ouate de cellulose.

La quantité importante de ouate de cellulose enrobant les embouts des bâtonnets offre un pouvoir d'absorption plus important qu'un bâtonnet ouaté conventionnel.

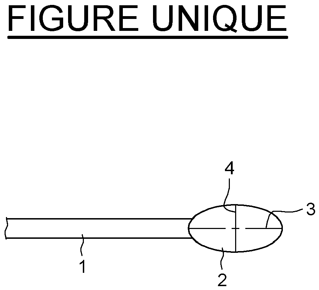

D'autres avantages et caractéristiques de l'invention apparaîtront à l'examen de la description détaillée d'un mode de réalisation, nullement limitatif, et du dessin annexé qui représente une vue en coupe longitudinale d'un embout de bâtonnet ouaté selon un mode de réalisation.

Dans le mode de réalisation illustré, le bâtonnet ouaté comprend un bâtonnet 1 comportant un embout 2 monté à l'une des extrémités du bâtonnet 1, et de préférence aux deux extrémités du bâtonnet. L'embout 1 possède une section longitudinale globalement ovale, en l'espèce en forme d'ellipse.

L'ellipse est définie par un grand axe 3 et un petit axe 4. Le grand axe 3 s'étend dans la direction du bâtonnet 1 tandis que le petit axe 4 s'étend orthogonalement au grand axe 3.

Le grand axe a par exemple une longueur de 12,5 mm. Le petit axe 4 possède ici une dimension de 6,4mm avec une tolérance de 0,7mm. Une telle dimension pour le petit axe 4, orthogonal à la direction d'introduction du bâtonnet ouaté dans l'oreille, et de manière générale le diamètre de l'embout, permet d'empêcher l'introduction de l'embout 2 du bâtonnet ouaté en profondeur dans le conduit auditif d'un usager.

En effet, une telle dimension transversale d'embout est supérieure à celle d'une zone interne du conduit auditif d'un utilisateur. Elle est en particulier supérieure à une section du conduit auditif situé immédiatement après un premier coude que forme le conduit, après le pavillon. On limite ainsi toute introduction profonde de l'embout 2 dans le conduit auditif. Il a ainsi été constaté que de telles dimensions permettaient d'éviter toute pénétration de l'embout au-delà du premier tiers du conduit. Toutefois, on ne sort pas du cadre de l'invention lorsque le diamètre de l'embout 2 présente des valeurs différentes, par exemple comprises entre environ 5 et 9 mm, et avantageusement entre 5,7 et 9mm, dans la mesure où de telles dimensions permettent d'éviter la pénétration profonde de l'embout dans le conduit auditif tout permettant un nettoyage efficace du pavillon de l'oreille.

Par ailleurs, l'embout 2 est réalisé avec de la ouate de cellulose. L'embout 2 comprend ici 82mg de ouate de cellulose avec une tolérance de 8mg. Bien entendu, on ne sort pas davantage du cadre de l'invention lorsqu'une quantité différente d'ouate est utilisée pour la réalisation de l'embout. On notera que la présence d'une telle quantité accrue d'ouate, par exemple comprise entre 60 mg et 120 mg permet d'augmenter les propriétés absorbantes du bâtonnet.

Il a en outre été constaté que la présence d'une telle quantité d'ouate permet d'absorber efficacement l'eau présente au niveau du pavillon et éviter ainsi que cette eau ne pénètre dans le conduit auditif.

A la fabrication, l'ouate de cellulose est enrobée autour de l'extrémité du bâtonnet 1 afin de former un embout 2 de forme généralement ovale ayant un axe transversale d'environ 6,4mm de diamètre, et ayant de l'ordre de 82 mg d'ouate de cellulose, offrant ainsi un pouvoir d'absorption plus important qu'un bâtonnet ouaté classique.

## Revendications

1. Bâtonnet ouaté comprenant un bâtonnet (1) et au moins un embout (2) monté à l'une des extrémités du bâtonnet (1), **caractérisé en ce que** l'embout (2) comporte au moins une dimension transversale supérieure à celle d'un conduit auditif d'un utilisateur de manière à limiter son insertion dans le conduit auditif.

2. Bâtonnet ouaté selon la revendication 1, dans lequel ladite dimension transversale est comprise entre 5 mm et 9 mm, et plus particulièrement entre 5,7 mm et 9 mm.

3. Bâtonnet ouaté selon la revendication 2, dans lequel l'embout a une section longitudinale généralement ovale définie par un grand axe (3) s'étendant selon l'axe du bâtonnet (1) et un petit axe (4) ayant ladite dimension comprise entre 5 mm et 9 mm, et plus particulièrement entre 5,7 mm et 9 mm.

4. Bâtonnet ouaté selon l'une quelconque des revendications 1 à 3, dans lequel les moyens d'absorption comprennent entre 60 mg et 120 mg de ouate de cellulose, et notamment 82 mg de ouate de cellulose.
